**Europäisches Patentamt**

**European Patent Office**

**Office européen des brevets**

(19)

(11) Veröffentlichungsnummer : **0 424 695 B1**

(12)

# EUROPÄISCHE PATENTSCHRIFT

(45) Veröffentlichungstag der Patentschrift :
**12.05.93 Patentblatt 93/19**

(51) Int. Cl.$^5$ : **C07C 37/62,** C07C 39/367,
C07C 37/84

(21) Anmeldenummer : **90118849.0**

(22) Anmeldetag : **02.10.90**

(54) Verfahren zur Herstellung von Tetrabrom-4,4'-alkyliden-diphenolen.

(30) Priorität : **23.10.89 DE 3935224**

(43) Veröffentlichungstag der Anmeldung :
**02.05.91 Patentblatt 91/18**

(45) Bekanntmachung des Hinweises auf die
Patenterteilung :
**12.05.93 Patentblatt 93/19**

(84) Benannte Vertragsstaaten :
**BE DE FR GB NL**

(56) Entgegenhaltungen :
**EP-A- 0 367 869**
**DE-A- 2 511 981**
**FR-A- 2 274 586**

(73) Patentinhaber : **Degussa Aktiengesellschaft**
**Weissfrauenstrasse 9**
**W-6000 Frankfurt am Main 1 (DE)**

(72) Erfinder : **Walter, Eberhard, Dr.**
**Schifferstrasse 88**
**W-6000 Frankfurt 70 (DE)**

## Beschreibung

Die Erfindung richtet sich auf ein verfahren zur Herstellung von Tetrabrom-4,4'-alkylidendiphenolen, worunter 4,4'-Alkyliden-bis-(2,6-dibromphenole) verstanden werden, insbesondere von 4,4'-Isopropyliden-bis-(2,6-dibromphenol), das auch als 2,2-Bis-(3,5-dibrom-4-hydroxyphenyl)-propan, aber meist als Tetrabrombisphenol A oder kurz TBBA bezeichnet wird. Das verfahren beruht auf der Bromierung der Alkylidendiphenole mit Brom in Gegenwart von Wasserstoffperoxid und ist durch eine Waschstufe gekennzeichnet. Das Verfahren ermöglicht es, die Mutterlauge zu rezyklieren und Tetrabrom-4,4'-alkylidendiphenole in hoher Ausbeute und hoher Qualität herzustellen.

Kernbromierte phenolische Verbindungen, wie Tetrabrombisphenol A (TBBA) und Tribromphenol, finden als Flammschutzmittel in Kunststoffen und Kunstharzen Anwendung. TBBA nimmt hierbei eine herausragende Stelle ein, weil das Flammschutzmittel über die beiden phenolischen Hydroxylgruppen reaktiv in Polymersysteme, etwa Epoxidharze und Polyester, eingebaut werden kann. Hierbei werden an die Qualität des TBBA bzw. anderer Tetrabrom-4,4'-alkylidendiphenole hohe Anforderungen gestellt: Die Produkte müssen möglichst frei von Nebenprodukten sein, eine möglichst niedrige Farbzahl aufweisen und praktisch frei von hydrolysierbarem Brom sein.

Marktgängige TBBA-Qualitäten weisen mitunter hohe APHA-Farbzahlen auf, so daß ihre Verwendung in insbesondere transparenten Produkten zu unerwünschten Verfärbungen führt. Zudem verursacht ihr ggf. hoher Anteil an aliphatisch gebundenem Brom Korrosion durch die hydrolytische oder thermische Abspaltung von Bromwasserstoffsäure und mindert die elektrischen Isolationswerte von mit solchem TBBA flammhemmend ausgerüstetem Kunstharzen. In vielen Fällen sind die Verarbeiter daher gezwungen, Standardqualitäten selbst zu reinigen oder Sonderqualitäten einzusetzen, was jeweils zu erhöhten Kosten führt.

Die Bromierung von 4,4'-Alkylidendiphenolen mit Brom zu den kernbromierten Tetrabrom-4,4'-alkylidendiphenolen ist lange bekannt. Die bei der Bromierung entstehende Bromwasserstoffsäure kann zu Brom oxidiert und somit für die Bromierung erneut verfügbar gemacht werden. Zur Reoxidation wurden Schwefeltrioxid oder Chlor verwendet, was aber wegen der Bildung von Schwefeldioxid bzw. Chlorwasserstoff mit erheblichen Umweltbelastungen einhergeht.

Ein einfaches und umweltfreundliches Verfahren, bei welchem Phenole und 4,4'-Alkylidendiphenole in Gegenwart von Wasserstoffperoxid bromiert werden, wurde in der US-PS 3,929,907 beschrieben. Bei diesem Verfahren, das auch die Grundlage des erfindungsgemäßen Verfahrens bildet, wird die bei der Bromierung entstehende Bromwasserstoffsäure in situ durch wäßrige Wasserstoffperoxidlösung zu Brom oxidiert, das dann für die weitere Bromierung zur Verfügung steht. Die Peroxobromierung von 4,4'-Alkylidendiphenolen, wie Bisphenol A, erfolgt bei 0 °C bis 100 °C in Gegenwart eines inerten organischen Lösungsmittels, das mit Wasser nicht mischbar ist, sich aber zur Kristallisation der Tetrabrom-4,4'-alkylidendiphenole eignet. Nach der Bromierung wird das Reaktionsgemisch soweit erwärmt, daß darin suspendiertes Reaktionsprodukt in der organischen Phase gelöst wird und die wäßrige Phase abgetrennt werden kann; anschließend wird die organische Phase abgekühlt wobei das bromierte Produkt auskristallisiert und dann in bekannter Weise isoliert werden kann.

Durch mehrfache Wiederverwendung der Mutterlauge des Verfahrens der US-PS 3,929,907 kann zwar die Ausbeute gesteigert werden, es zeigte sich hierbei jedoch, daß gleichzeitig die Qualität des Tetrabrom-4,4'-alkylidendiphenols zunehmend gemindert wird. Besonders deutlich wird dies an der APHA-Farbzahl und dem Gehalt an verseifbarem Brom, welche sich bereits nach einmaliger Rezyklierung der Mutterlauge etwa verdoppeln. Bei der im Betriebsmaßstab durchgeführten vorbekannten Peroxobromierung von Bisphenol A zu TBBA unter Verwendung von Chlorbenzol als Lösungsmittel weist das TBBA ohne Mutterlaugerückführung eine APHA-Farbzahl um 150 und einen verseifbaren Bromidanteil um 600 ppm auf; diese Qualität konnte in Folgeansätzen nur dann aufrechterhalten werden, wenn zuvor die Mutterlauge durch Redestillation von qualitätsmindernden Stoffen befreit wurde. Eine Redestillation der gesamten Mutterlauge ist sehr aufwendig und verteuert das Verfahren.

Aufgabe der Erfindung ist, das vorbekannte Verfahren der Peroxobromierung zur Herstellung von Tetrabrom-4,4'-alkylidendiphenolen, insbesondere von TBBA, aus den entsprechenden 4,4'-Alkylidendiphenolen so zu verbessern, daß auch bei mehrfacher Rezyklierung der Mutterlauge die qualitätsbestimmenden Merkmale des bromierten Produkts weitgehend Konstant bleiben. Eine weitere Aufgabe richtet sich darauf, die Qualität des Produktes über das bisher durch Peroxobromierung erreichte Niveau zu steigern, ohne die Ausbeute zu mindern und ohne den Gesamtaufwand für das Verfahren nennenswert zu erhöhen.

Gelöst wird diese Aufgabe durch ein Verfahren zur Herstellung von Tetrabrom-4,4'-alkylidendiphenolen hoher Reinheit durch Bromieren des entsprechenden 4,4'-Alkylidendiphenols mit Brom oder einer wäßrigen HBr$_3$- oder HBr-Lösung, wobei eingesetztes und bei der Bromierung gebildetes HBr durch wäßriges Wasserstoffperoxid zu Brom oxidiert wird, in Gegenwart eines mit Wasser nicht mischbaren organischen Lösungsmittels oder Lösungsmittelgemisches, worin das gebildete Tetrabrom-4,4'-alkylidendiphenol nach beendeter Umsetzung durch Erhöhung der Temperatur des Reaktionsgemischs gelöst wird, Abtrennung der wäßrigen von der organischen Phase, Abkühlung der organischen Phase, wobei das Tetrabrom-4,4'-alkylidendiphenol kristallisiert, und Abtrennung der Kristalle von der Mutterlauge, das dadurch gekennzeichnet ist, daß man die organische Phase vor der Kristallisation mindestens einmal mit einer wäßrigen Alkalisulfitlösung und mindestens einmal mit Wasser wäscht und nach jeder Wäsche die wäßrige Phase abtrennt.

Die Unteransprüche richten sich auf bevorzugte Ausführungsformen des Verfahrens.

Entsprechend der beispielhaften Ausführungsform der US-PS 3,929,907 kann zu einer Mischung aus dem 4,4'-Alkylidendiphenol und dem organischen Lösungsmittel zunächst Brom und im Anschluß daran eine wäßrige Wasserstoffperoxidlösung zudosiert werden. Erfindungsgemäß wird jedoch die umgekehrte Reihenfolge bevorzugt, d. h., das Brom wird zu einem Gemisch aus dem im allgemeinen suspendiert vorliegenden 4,4'-Alkylidendiphenol, dem organischen Lösungsmittel und dem wäßrigen Wasserstoffperoxid geregelt zudosiert. Eine Temperaturbegrenzung während der Bromzugabe auf 40 °C, vorzugsweise 30 °C, hat sich als vorteilhaft erwiesen; infolge der hohen Reaktionswärme muß hierbei gekühlt werden. Anstelle Brom können auch wäßrige Lösungen von Bromwasserstoffsäure oder konzentrierte Lösungen von Brom in Bromwasserstoffsäure ($HBr_3$) zur Bromierung eingesetzt werden.

Das 4,4'-Alkylidendiphenol, Brom und Wasserstoffperoxid werden vorzugsweise im Molverhältnis 1 zu 2 bis 2,1 zu 2 bis 2,2 eingesetzt. Im Falle HBr-haltiger Bromierungssysteme wird pro Mol HBr und $Br_2$ je ein 1 bis 1,1 Mol Wasserstoffperoxid verwendet. Es ist zwar möglich, einen höheren Überschuß an Wasserstoffperoxid zu verwenden, jedoch führt dies nicht zu erkennbaren Verfahrens- und/oder Qualitätsvorteilen. Ein Überschuß von etwa 3 % Brom und etwa 5 % Wasserstoffperoxid ist im allgemeinen völlig ausreichend.

Die Alkylidengruppe der zu bromierenden 4,4'-Alkylidendiphenole kann 1-8 C-Atome enthalten, wobei Strukturen mit einer tertiären C-H-Bindung ausgeschlossen sind. Die Isopropylidengruppe wird bevorzugt.

Kennzeichen des erfindungsgemäßen Verfahrens ist eine in den an sich bekannten Verfahrensablauf integrierte Wäsche der organischen Phase, welche das bei der Bromierung gebildete Tetrabrom-4,4'-alkylidenphenol gelöst enthält, mit einer wäßrigen Alkalisulfitlösung. Um einen guten Wascheffekt zu erzielen, wird die Wäsche bei erhöhter Temperatur, vorzugsweise bei 50 bis 90 °C, insbesondere bei 70 bis 90 °C, durchgeführt. Die Waschbehandlung ist mindestens einmal erforderlich, jedoch kann diese nach jeweils erfolgter Phasentrennung auch mehrmals wiederholt werden. Unter den Alkalisulfiten, welche zur Anwendung gelangen, wird Natriumsulfit bevorzugt. Die Konzentration der wäßrigen Alkalisulfitlösung kann den gesamten Konzentrationsbereich bis zur Sättigungsgrenze bei der gewählten Behandlungstemperatur überstreichen; im allgemeinen wird die Konzentration im Bereich von 1 bis 20 Gew.-%, insbesondere 5 bis 15 Gew.-%, liegen. Gemäß einer bevorzugten Ausführungsform werden zur Waschbehandlung einer bei 50 bis 90 °C gesättigten Lösung von in Chlorbenzol pro kg TBBA 25 bis 100 g $Na_2SO_3$ in Form einer wäßrigen, vorzugsweise 10 bis 15 gew.-%igen, Lösung verwendet; eine Waschstufe reicht im allgemeinen aus. Die Waschbehandlung kann in einfachen Rührreaktoren oder in Waschkolonnen oder anderen Apparaten, welche eine ausreichende Durchmischung der beiden Phasen und ggf. Aufrechterhaltung der gewünschten Behandlungstemperatur gewährleisten, wie etwa Mixer-Settler-Einheiten erfolgen.

Je nach Intensität der Waschbehandlung und der Temperatur, bei welcher sie durchgeführt wird, muß mit einer Kontaktzeit im Bereich von 1 bis 60 Minuten gerechnet werden. Bei der besonders bevorzugten Behandlungstemperatur im Bereich von 70 Bis 90 °C ist im allgemeinen eine Kontaktzeit von 5 bis 20 Minuten ausreichend. Nach der Waschbehandlung der organischen Phase mit einer wäßrigen Alkalisulfitlösung schließt sich mindestens eine Nachwäsche mit Wasser an, wobei auch hierbei eine Temperatur gewählt wird, bei welcher das gelöste Tetrabrom-4,4'-alkylidendiphenol noch nicht auskristallisiert. Um den Wasserhaushalt im Verfahren zu begrenzen, ist es vorteilhaft, das Waschwasser einem nachfolgenden Ansatz zur Herstellung der wäßrigen Alkalisulfitlösung zuzuführen. Die gebrauchten Waschlösungen lassen sich in einfacher Weise dadurch entsorgen, daß Sulfit zu Sulfat oxidiert wird, beispielsweise mit Wasserstoffperoxid, und dabei ausfallende Bromphenole abfiltriert werden; alternativ können nach der Oxidation die bromorganischen Bestandteile auch durch eine Extraktion mit dem in der Bromierung eingesetzten organischen Lösungsmittel aus dem Abwasser entfernt werden.

Nach der erfindungsgemäßen Waschbehandlung und letzten Phasentrennung läßt man die organische Phase abkühlen, wobei das gewünschte Tetrabrom-4,4'-alkylidendiphenol auskristallisiert. Die Durchführung der Kristallisation und Trennung der Kristalle von der Mutterlauge erfolgt nach den allgemein bekannten Methoden und unter Verwendung üblicher Fest-Flüssig-Trennorgane.

Die Bromierung erfolgt in Gegenwart eines gegenüber den Reaktionspartnern inerten organischen Lösungsmittels, aus welchem die Tetrabromverbindung auskristallisiert werden kann. Das verwendete Lösungsmittel soll somit einen deutlichen Konzentrations-Temperaturgradienten für die Tetrabromverbindung aufweisen. Besonders vorteilhaft ist in dieser Hinsicht Chlorbenzol - hier beträgt die Sättigungskonzentration an TBBA bei 20 °C 185 g/l, bei 90 °C 805 g/l. Andere Lösungsmittel sind die in der US-PS 3,929,907 genannten, wie etwa Benzol, halogenierte aliphatische Kohlenwasserstoffe, 2-Ethylhexanol in Kombination mit aliphatischen Kohlenwasserstoffen. Lösungsmittel mit einem Siedepunkt im Bereich von etwa 80 bis 200 °C, insbesondere etwa 80 bis 150 °C, werden bevorzugt. Das zu bromierende Phenol kann sich in dem Lösungsmittel ganz oder teilweise lösen, es kann jedoch auch als darin suspendierter Feststoff umgesetzt werden, wie dies bei der Oxobromierung von Bisphenol A zu TBBA in Chlorbenzol der Fall ist.

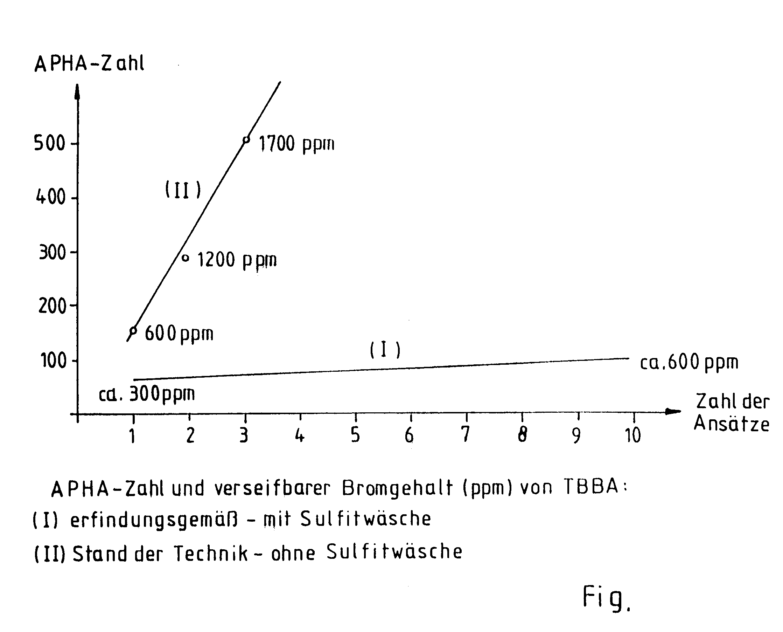

Durch die erfindungsgemäße Wäsche mit wäßriger Alkalisulfitlösung ist es überraschenderweise möglich, den größten Teil der Mutterlauge nach der Kristallisation und Filtration ohne Destillation in das Verfahren zurückzuführen. Es genügt, jeweils nur einen kleinen Teilstrom, im allgemeinen etwa 5 bis 10 %, der Mutterlauge auszuschleusen und aufzuarbeiten; hierbei wird der entsprechende Teil des Lösungsmittels zurückgewonnen, und aus dem Destillationssumpf kann 2,4,6-Tribromphenol, das selbst ein Flammschutzmittel ist, gewonnen werden. Neben der Rezyklierbarkeit der Mutterlauge wird gleichzeitig die Qualität des gewonnenen Tetrabrom-4,4'-alkylidendiphenols bezüglich der Farbzahl und des verseifbaren Brom-Gehalts verbessert, was nicht vorhersehbar war. Figur 1/1 zeigt am Beispiel von aufeinanderfolgenden Ansätzen zur Herstellung von TBBA im Technikumsmaßstab die gefundenen Werte für die genannten Qualitätsmerkmale von nach dem erfindungsgemäßen Verfahren hergestelltem TBBA - Kurve (I) - sowie, zum Vergleich, von nach dem Stand der Technik hergestelltem TBBA - Kurve (II). Sowohl bei den Ansätzen gemäß (I) als auch (II) wurde unter gleichen Bedingungen oxobromiert und kristallisiert und jeweils die gesamte Mutterlauge in den Folgeansatz rezykliert. Die Überlegenheit des erfindungsgemäßen Verfahrens wird durch Figur 1/1 offensichtlich.

Durch die erfindungsgemäße Wäsche mit einer wäßrigen Sulfitlösung werden farbgebende Nebenprodukte mit vermutlich chinoider Struktur in farblose Verbindungen überführt und Nebenprodukte mit aliphatisch gebundenem Brom zum Teil hydrolysiert und zum Teil mit der Waschphase aus dem System ausgetragen. Durch die erfindungsgemäße Maßnahme werden ein qualitativ besseres TBBA erzeugt und die Nebenprodukte unschädlich und damit die Mutterlauge problemlos rezyklierbar gemacht.

Beispiel 1: Reihenversuch zur Herstellung von TBBA aus Bisphenol A und Brom/$H_2O_2$

In einem emaillierten Rührbehälter wird Chlorbenzol zusammen mit einer wäßrigen Lösung von Wasserstoffperoxid und Bisphenol A vorgelegt. Zur Reaktion wird in die gut durchmischte Suspension Brom zudosiert. Die Reaktionswärme wird durch Solekühlung abgeführt. Die Bromzugabe wird so gesteuert, daß eine maximale Temperatur von 30 °C nicht überschritten wird. Das zudosierte Brom reagiert rasch. Nach der Bromzugabe wird die Suspension auf 80 °C aufgeheizt, wobei das gebildete TBBA quantitativ in Lösung geht. Die wäßrige Phase wird von der organischen Phase getrennt. Die das TBBA gelöst enthaltende organische Phase wird einmal mit einer 1-molaren wäßrigen Natriumsulfitlösung - 250 ml pro Mol TBBA - und zweimal mit Wasser bei etwa 80 °C durch intensives Mischen gewaschen; nach jedem Waschvorgang wird die wäßrige Phase abgehebert. Zur Kristallisation wird die organische Phase langsam auf 20 °C abgekühlt. Die entstandene Feststoffsuspension wird auf einer Zentrifuge getrennt. Das Feuchtprodukt, es enthält 5-10 % Chlorbenzol, wird in üblicher Weise getrocknet.

Pro Mol Bisphenol A wurden 2,1 Mol 50 gew.-%iges wäßriges Wasserstoffperoxid, das 3 gew.-%ig mit $H_2SO_4$ sauer eingestellt war, und 2.06 Mol Brom eingesetzt; pro Mol Bisphenol A wurden im 1. Ansatz 800 ml Chlorbenzol vorgelegt.

Die Mutterlauge wird ohne Ersatz der Lösungsmittelverluste im Folgeansatz eingesetzt, wobei die Menge an Reaktionspartnern in den Folgeansätzen im gleichen Verhältnis reduziert wurden.

Der Tabelle 1 sind die Ergebnisse von 10 aufeinanderfolgenden Versuchen zu entnehmen. Die Gesamtausbeute an TBBA atro betrug 96,4 % d. Th., wobei die Mutterlauge nach dem 10. Ansatz weitere 124 g TBBA entsprechend 1,6 % d. Th. sowie 100 g Tribromphenol enthielt.

## Tabelle 1

| Versuch Nr. | Einsatz Bisphenol A (g) | TBBA (trocken) (g) | Mutterlauge (ml) | APHA-Zahl des TBBA *) | Verseifbarer Bromid-gehalt **) des TBBA (ppm) |
|---|---|---|---|---|---|
| 1 | 570 | 1082 | 1935 | 10 | 300 |
| 2 | 428 | 1032 | 1760 | 10 | 300 |
| 3 | 377 | 886 | 1600 | 10 | 300 |
| 4 | 343 | 833 | 1500 | 30 | 360 |
| 5 | 321 | 755 | 1370 | 40 | 430 |
| 6 | 293 | 705 | 1270 | 40 | 480 |
| 7 | 272 | 643 | 1170 | 50 | 520 |
| 8 | 250 | 603 | 1060 | 50 | 520 |
| 9 | 227 | 528 | 1000 | 70 | 580 |
| 10 | 212 | 516 | 920 | 60 | 590 |

*)  Bestimmung nach DIN 53 409, wobei 50 ml einer 50 gew.-%igen Lösung von TBBA in Aceton gegen $[Co\ PtCl_6]$-Standard-Lösungen verglichen wurden.

**)  Bestimmung durch einstündiges Kochen von 100 g TBBA mit einer 10 gew.-%igen NaOH-Lösung, Neutralisation, Abfiltrieren des TBBA und Nachwaschen, Bestimmung des Bromidgehaltes.

EP 0 424 695 B1

Beispiel 2: Reihenversuch zur Herstellung von TBBA aus Bisphenol A und HBr/H$_2$O$_2$

Der Verfahrensablauf entsprach demjenigen des Beispiels 1, jedoch wurde anstelle mit Brom/H$_2$O$_2$ mit konz. wäßrigem HBr/H$_2$O$_2$ oxobromiert. Molverhältnis Bisphenol A : HBr : H$_2$O$_2$ = 1 : 4,12 : 4,2. Ferner wurde im 1. Ansatz das Chlorbenzol (1000 ml) mit 225 g TBBA vorgesättigt. Um dem mit dem gebildeten TBBA ausgetragenen Chlorbenzol Rechnung zu tragen, wurden ab dem 2. Ansatz jeweils 100 ml frisches Chlorbenzol hinzugefügt ; in 10 aufeinanderfolgenden Versuchen mit vollständiger Rückführung der Mutterlauge wurde jeweils 1 Mol Bisphenol A eingesetzt, umgesetzt und erfindungsgemäß wie in Beispiel 1 gewaschen. Die Ergebnisse folgen aus der Tabelle 2. Die Gesamtausbeute an TBBA atro betrug 94,5 % d. Th. einschließlich TBBA aus der Versättigung, ferner waren 2,5 % d. Th. in der Mutterlauge des 10. Ansatzes enthalten.

## Tabelle 2

| Versuch Nr. | TBBA (trocken) (g) | Mutterlauge (ml) | TBBA-Stoffdaten | |
|---|---|---|---|---|
| | | | Schmelzpunkt (°C) | APHA-Zahl |
| 1 | 603 | 1310 | 181 - 182 | 10 |
| 2 | 494 | 1300 | 180 - 182 | 20 |
| 3 | 513 | 1300 | 180 - 182 | 20 |
| 4 | 559 | 1280 | 180 - 182 | 20 |
| 5 | 497 | 1300 | 179 - 182 | 30 |
| 6 | 533 | 1260 | 179 - 182 | 20 |
| 7 | 478 | 1300 | 179 - 180 | 20 |
| 8 | 537 | 1290 | 178 - 181 | 20 |
| 9 | 590 | 1260 | 179 - 181 | 30 |
| 10 | 551 | 1220 | 179 - 181 | 30 |

EP 0 424 695 B1

**Patentansprüche**

1.  Verfahren zur Herstellung von Tetrabrom-4,4'-alkylidendiphenolen hoher Reinheit durch Bromieren des entsprechenden 4,4'-Alkylidendiphenols mit Brom oder einer wäßrigen $HBr_3$- oder HBr-Lösung, wobei eingesetztes und bei der Bromierung gebildetes HBr durch wäßriges Wasserstoffperoxid zu Brom oxidiert wird, in Gegenwart eines mit Wasser nicht mischbaren organischen Lösungsmittels oder Lösungsmittelgemisches, worin das gebildete Tetrabrom-4,4'-alkylidendiphenol nach beendeter Umsetzung durch Erhöhung der Temperatur des Reaktionsgemisches gelöst wird, Abtrennung der wäßrigen von der organischen Phase, Abkühlung der organischen Phase, wobei das Tetrabrom-4,4'-alkylidendiphenol kristallisiert, und Abtrennung der Kristalle von der Mutterlauge,
    dadurch gekennzeichnet,
    daß man die organische Phase vor der Kristallisation mindestens einmal mit einer wäßrigen Alkalisulfitlösung und mindestens einmal mit Wasser wäscht und nach jeder Wäsche die wäßrige Phase abtrennt.

2.  Verfahren nach Anspruch 1,
    dadurch gekennzeichnet,
    daß man die organische Phase bei 50 - 90 °C, vorzugsweise bei 70 - 90 °C, mit einer 1 bis 20 gew.-%igen, vorzugsweise 5 bis 15 gew.-%igen, wäßrigen Natriumsulfitlösung wäscht.

3.  Verfahren nach Anspruch 1 oder 2,
    dadurch gekennzeichnet,
    daß man 4,4'-Isopropylidendiphenol, Brom und Wasserstoffperoxid im Molvernältnis 1 zu 2 bis 2,1 zu 2 bis 2,2 einsetzt.

4.  Verfahren nach Anspruch 3,
    dadurch gekennzeichnet,
    daß man pro kg gebildetem Tetrabrom-4,4'-isopropylidendiphenol 25 bis 100 g Natriumsulfit in Form einer 10 bis 15 gew.-%igen wäßrigen Lösung zur Wäsche einsetzt.

5.  Verfahren nach Anspruch 3 und/oder 4,
    dadurch gekennzeichnet,
    daß man ein Gemisch aus 4,4'-Isopropylidendiphenol, Lösungsmittel und wäßrigem Wasserstoffperoxid in einem Reaktor vorlegt und unter intensivem Durchmischen des Reaktionsgemisches Brom zudosiert, wobei die Temperatur auf 40 °C, vorzugsweise 30 °C, begrenzt wird.

6.  Verfahren nach einem oder mehreren der Ansprüche 1 bis 5,
    dadurch gekennzeichnet,
    daß man 90 bis 100 % der Mutterlauge ohne weitere Reinigung rezykliert.

7.  Verfahren nach einem oder mehreren der Ansprüche 1 bis 6,
    dadurch gekennzeichnet,
    daß man Chlorbenzol als Lösungsmittel verwendet.


**Claims**

1.  Process for the production of tetrabromo-4,4'- alkylidenediphenols of high purity by bromination of the corresponding 4,4'-alkylidenediphenol with bromine or an aqueous $HBr_3$ or HBr solution, HBr charged and formed during the bromination being oxidized to bromine by aqueous hydrogen peroxide, in the presence of a water-immiscible organic solvent or solvent mixture, in which after completion of the reaction the tetrabromo-4,4'-alkylidenediphenol formed is dissolved by raising the temperature of the reaction mixture, separating the aqueous from the organic phase, cooling the organic phase, during which the tetrabromo-4,4'-alkylidenediphenol crystallizes, and separating the crystals from the mother liquor,
    characterized in that the organic phase is washed before the crystallization at least once with an aqueous alkali sulphite solution and at least once with water and after each wash the aqueous phase is removed.

2.  Process according to Claim 1,
    characterized in that
    the organic phase is washed at 50-90 °C, preferably at 70-90 °C, with a 1-20 % by weight, preferably 5-15 % by weight aqueous sodium sulphite solution.

3. Process according to Claim 1 or 2,
characterized in that
4,4'-isopropylidenediphenol, bromine and hydrogen peroxide are used in the molar ratio 1 to 2-2.1 to 2-2.2.

4. Process according to Claim 3,
characterized in that
25 to 100 g sodium sulphite in the form of a 10 to 15 wt% aqueous solution per kg tetrabromo-4,4'-isopropylidenediphenol formed are used for the washing.

5. Process according to Claim 3 and/or 4,
characterized in that
a mixture of 4,4'-isopropylidenediphenol, solvent and aqueous hydrogen peroxide is charged to a reactor and bromine is metered in with intense, thorough mixing of the reaction mixture, the temperature being limited to 40 °C, preferably 30 °C.

6. Process according to one or more of Claims 1 to 5,
characterized in that
90 to 100 % of the mother liquor is recycled without further purification.

7. Process according to one or more of Claims 1 to 6,
characterized in that
chlorobenzene is used as solvent.

## Revendications

1. Procédé de préparation de tétrabromo-4,4'-alkylidènediphénols de haute pureté par bromation du 4,4'-alkylidènediphénol corrrespondant avec du brome ou une solution aqueuse de HBr$_3$- ou une solution aqueuse de HBr, le HBr utilisé ou formé lors de la bromation est oxydé en brome par le peroxyde d'hydrogène aqueux, en présence d'un solvant organique non miscible à l'eau, ou un mélange de tels solvants, dans lequel on dissout le tétrabromo-4,4'-alkylidènediphénol formé, à la fin de la réaction par augmentation de la température du mélange réactionnel, séparation de la phase aqueuse de la phase organique, refroidissement de la phase organique, ce qui fait cristalliser le tétrabromo-4,4'-alkylidènediphénol, et séparation des cristaux de la solution mère, procédé caractérisé en ce qu'on lave la phase organique avant la cristallisation au moins une fois avec une solution aqueuse de sulfite alcalin, et au moins une fois à l'eau, et qu'après chaque lavage on sépare la phase aqueuse.

2. Procédé selon la revendication 1, caractérisé en ce qu'on lave la phase organique à 50-90°C, de préférence à 70-90°C, avec une solution aqueuse de sulfite de sodium de 1 à 20 % en poids, de préférence de 5 à 15 % en poids.

3. Procédé selon la revendication 1 ou 2, caractérisé en ce qu'on utilise du 4,4'-isopropylidènediphénol, du brome et du peroxyde d'hydrogène en proportions molaires 1 à 2 jusqu'à 2,1, à 2 jusqu'à 2,2.

4. Procédé selon la revendication 3, caractérisé en ce qu'on utilise pour le lavage, par kg de tétrabromo-4,4'-isopropylidènediphénol formé, 25 à 100 g de sulfite de sodium sous forme d'une solution de 10 à 15 % en poids.

5. Procédé selon la revendication 3 et/ou 4, caractérisé en ce qu'on place dans un réacteur un mélange de 4,4'-isopropylidènediphénol, du solvant et de la solution aqueuse de peroxyde d'hydrogène et qu'en mélangeant énergiquement le mélange réactionnel, on ajoute en dosant le brome, en limitant la température à 40°C, de préférence 30°C.

6. Procédé selon l'une ou plusieurs des revendications 1 à 5, caractérisé en ce qu'on recycle 90 à 100 % de la solution mère sans autre purification.

7. Procédé selon l'une ou plusieurs des revendications 1 à 6, caractérisé en ce qu'on utilise le chlorobenzène comme solvant.

APHA-Zahl und verseifbarer Bromgehalt (ppm) von TBBA:

(I) erfindungsgemäß - mit Sulfitwäsche

(II) Stand der Technik - ohne Sulfitwäsche

Fig.

EP 0 424 695 B1